# EUROPÄISCHE PATENTSCHRIFT

(11) **EP 0 440 762 B1**
(45) Veröffentlichungstag und Bekanntmachung des Hinweises auf die Patenterteilung: **17.07.1996**
(21) Anmeldenummer: 90912004.0
(22) Anmeldetag: 17.08.1990
(51) Int. Cl.: C07D 213/65, C09K 19/34

(54) **5-OXY-2-PHENYLPYRIDINE UND FLÜSSIGKRISTALLINES MEDIUM**
5-OXY-2-PHENYLPYRIDINES AND LIQUID-CRYSTAL MATERIALS
5-OXY-2-PHENYLPYRIDINE ET MILIEU DE CRISTAUX LIQUIDES

(30) Priorität: 26.08.1989 DE 3928264
(43) Veröffentlichungstag der Anmeldung: 14.08.1991
(73) Patentinhaber: HOECHST AKTIENGESELLSCHAFT, 65926 Frankfurt am Main (DE)
(72) Erfinder: WÄCHTLER, Andreas, D-6103 Griesheim (DE); REIFFENRATH, Volker, D-6101 Rossdorf (DE); HITTICH, Reinhard, D-6101 Modautal 1 (DE); GEELHAAR, Thomas, D-6500 Mainz (DE); POETSCH, Eike, D-6109 Mühltal 6 (DE)
(86) Internationale Anmeldenummer: EP9001351
(87) Internationale Veröffentlichungsnummer: WO9102722

(56) Entgegenhaltungen:
- EP-A- 0 293 764
- GB-A- 2 161 808
- US-A- 4 876 026
- Patent Abstracts of Japan, unexaminated applications, C-field, vol. 12, 17. November 1988; & JP-A-63 165 344

## Beschreibung

Die Erfindung betrifft ein ferroelektrisches flüssigkristallines Medium mit mindestens zwei flüssigkristallinen Komponenten, dadurch gekennzeichnet, daß es ein oder mehrere 5-Oxy-2-phenlpyridine der Formel I enthält, 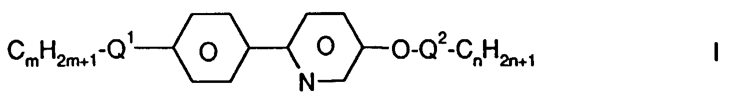 worin
- m und n: jeweils unabhängig voneinander 1 bis 18,
- Q¹: -O-, trans-1 ,4-Cyclohexylen, 1,4-Phenylen oder eine Einfachbindung und
- Q²: 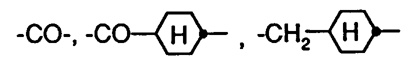 oder eine Einfachbindung bedeutet,
mit den Maßgaben, daß a) im Falle Q¹ = Q² = Einfachbindung die Summe von m + n ≥ 11 ist, und b) im Falle Q² = CO der Rest-CₙH₂ₙ₊₁ geradkettig ist.

Chirale getiltete smektische flüssigkristalline Medien mit ferroelektrischen Eigenschaften können hergestellt werden, indem man Basis-Mischungen mit einer oder mehreren getilteten smektischen Phasen mit einem geeigneten chiralen Dotierstoff versetzt (L.A. Beresnev et al., Mol. Cryst. Liq. Cryst. 89, 327 (1982); H.R. Brand et al., J. Physique 44 (lett.), L 771 (1983). Solche Medien können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften des chiralen getilteten Mediums beruhen. In diesen Medien sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1-2 µm) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiralen getilteten smektischen Phasen (wie z.B. Sc*, jedoch auch S_{H}*, S_{I}*, S_{J}*, S_{K}*, S_{G}*, S_{F}*) ist deren geringe chemische, thermische und Photo-Stabilität. Eine weitere nachteilige Eigenschaft von Displays basierend auf derzeit verfügbaren chiralen getilteten smektischen Medien ist, daß die Spontanpolarisation zu kleine Werte aufweist, so daß das Schaltzeitverhalten der Displays ungünstig beeinflußt wird und/oder der Pitch und/oder der Tilt und/oder die Viskosität der Medien nicht den Anforderungen der Display-Technologie entspricht. Darüberhinaus ist meist der Temperaturbereich der ferroelektrischen Medien zu klein und liegt überwiegend bei zu hohen Temperaturen.

Es wurde nun gefunden, daß die Verwendung von Verbindungen der Formel I als Komponenten chiraler getilteter smektischer Medien die erwähnten Nachteile wesentlich vermindern kann. Die Verbindungen der Formel I sind somit als Komponenten chiraler getilteter smektischer flüssigkristalliner Medien vorzüglich geeignet. Insbesondere sind mit ihrer Hilfe chemisch besonders stabile chirale getiltete smektische flüssigkristalline Medien mit günstigen ferroelektrischen Phasenbereichen, günstigen Werten für die Viskosität, insbesondere mit breiten Sc* Phasenbereichen, hervorragender Unterkühlbarkeit bis zu Temperaturen unter 0 °C ohne daß Kristallisation auftritt und für derartige Phasen hohen Werten für die spontane Polarisation herstellbar. P ist die spontane Polarisation in nC/cm². Die Verbindungen der Formel I eignen sich jedoch auch für flüssigkristalline Medien für den elektroklinen Effekt.

Die Verbindungen der Formel I besitzen einen breiten Anwendungsbereich. In Abhängigkeit von der Auswahl der Substituenten können diese Verbindungen als Basismaterialien dienen, aus denen flüssigkristalline smektische Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die Viskosität und/oder die spontane Polarisation und/ oder den Phasenbereiche und/oder der Tiltwinkel und/ oder den Pitch eines solchen Mediums zu variieren.

In der GB 21 61 808 ist eine sehr breite allgemeine Formel für nematische Phenylpyridine zur Anwendung in Multiplex-Zellen angegeben, die teilweise die hier beanspruchten Verbindungen der Formel I umfaßt. In der GB 21 61 808 sind jedoch keinerlei Hinweise für Sc-Phasen bei Verbindungen dieses Typs, vielmehr sollen die dort beschriebenen Verbindungen nematisch sein und eine excellente Mischbarkeit mit anderen nematischen Flüssigkristallen aufweisen. Im einzelnen sind folgende Verbindungen angegeben:
2-(p-Pentylphenyl)-5-butyloxypyridin, C→I 38 °C, I→S 34 °C
2-(p-Butylphenyl)-5-ethoxypyridin, C→I 29,5 °C
2-(p-Pentylphenyl)-5-propyloxypyridin, C→I 42 °C
2-(p-Hexylphenyl)-5-butyloxypyridin, C→S 26 °C, S→I 44,5 °C

Im Hinblick auf die gute Mischbarkeit mit anderen nematischen Flüssigkristallen können die bei zwei dieser Verbindungen beschriebenen, nicht identifizierten smektischen Phasen nicht S_{C}, sondern beispielsweise S_{B} sein.

Aus der JP 63 165 344 sind optisch aktive Phenylpyridine der Formel 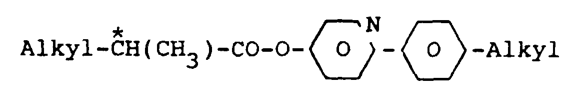 bekannt.

Diese eignen sich jedoch nicht als achirale Basismaterialien für ferroelektrische flüssigkristalline Medien, sondern werden als Dotierstoffe eingesetzt.

Der Fachmann konnte somit aus dem Stand der Technik nicht erkennen, daß die erfindungsgemäßen Verbindungen überwiegend breite und günstig gelegene Sc-Phasen aufweisen sowie sich durch günstige Werte für die Rotationsviskosität auszeichnen.

Gegenstand der Erfindung sind ferroelektrische flüssigkristalline Medien mit einem Gehalt an mindestens einer Verbindung der Formel I sowie Flüssigkristallanzeigen, insbesondere ferroelektrische elektrooptische Anzeigen, die derartige Medien enthalten.

Gegenstand der Erfindung sind ferner 5-Oxy-2-phenylpyridine gekennzeichnet durch die Formel Ib bis If: 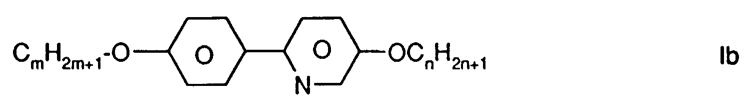 worin n und m jeweils unabhängig voneinander 5 bis 12 bedeuten. 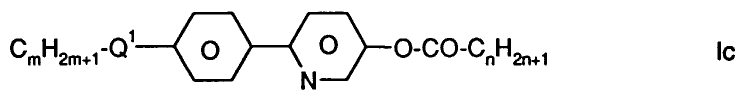 worin der Rest CₙH₂ₙ₊₁ geradkettig ist, und Q¹ -O- oder eine Einfachbindung und m und n jeweils unabhängig voneinander 5 bis 12 bedeuten; 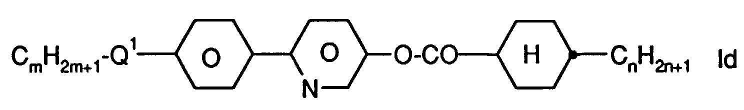 worin Q¹ -O- oder eine Einfachbindung, m 5 bis 12 und n 2 bis 12 bedeutet; 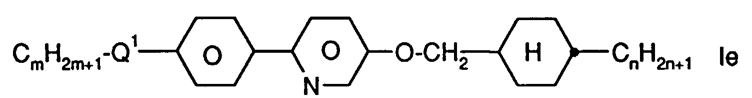 worin Q¹ -O- oder eine Einfachbindung, m 5 bis 12 und n 2 bis 12 bedeutet; 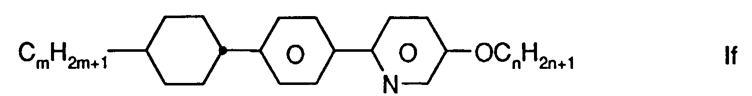 worin m und n jeweils unabhängig voneinander 1 bis 12 bedeuten.

Die erfindungsgemäßen Medien enthalten vorzugsweise mindestens zwei, insbesondere mindestens drei Verbindungen der Formel I. Besonders bevorzugt sind erfindungsgemäße chirale getiltete smektische flüssigkristalline Phasen, deren achirale Basismischung neben Verbindungen der Formel I mindestens eine andere Komponente mit negativer oder betragsmäßig kleiner positiver dielektrischer Anisotropie enthält. Diese weiteren Komponente(n) der achiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Formel IV, welche die Verbindungen der Teil formeln IVa bis IVi umfaßt:

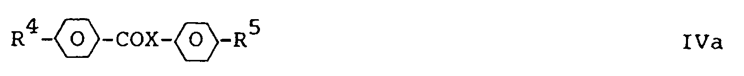

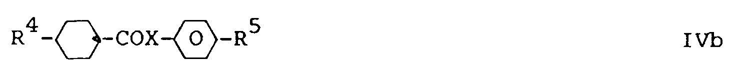

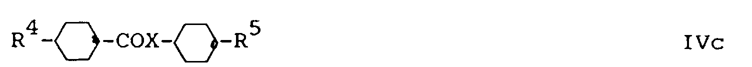

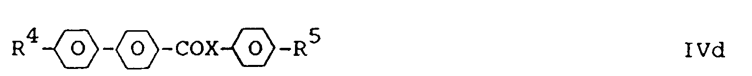

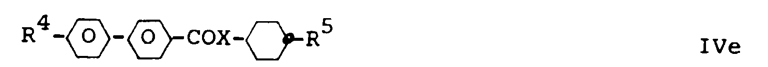

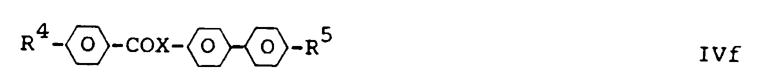

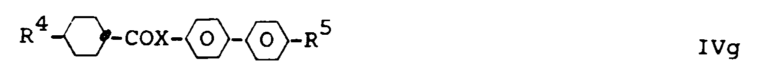

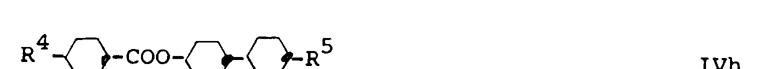

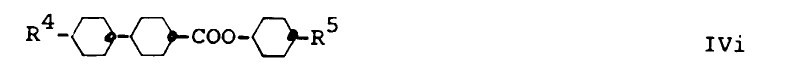

R⁴ und R⁵ sind jeweils vorzugsweise geradkettig Alkyl, Alkoxy, Alkanoyloxy oder Alkoxycarbonyl mit jeweils 3 bis 12 C-Atomen. X ist vorzugsweise O. In den Verbindungen der Formeln IVa, IVb, IVd, IVe, IVf und IVg kann auch eine 1,4-Phenylengruppe lateral durch Halogen oder CN, insbesondere bevorzugt durch Fluor, substituiert sein.

Besonders bevorzugt sind die Verbindungen der Teilformeln IVa, IVb, IVd und IVf, worin R⁴ und R⁵ jeweils geradkettiges Alkyl oder Alkoxy mit jeweils 5 bis 10 C-Atomen bedeutet.

Besonders bevorzugte Einzelverbindungen sind in der folgenden Tabelle I angegeben:

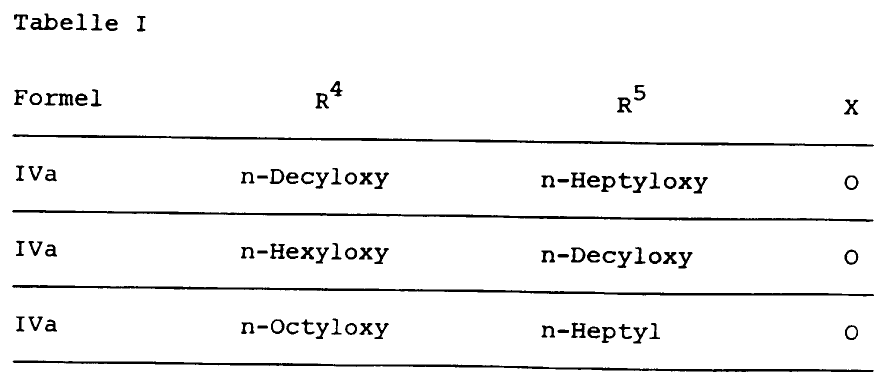

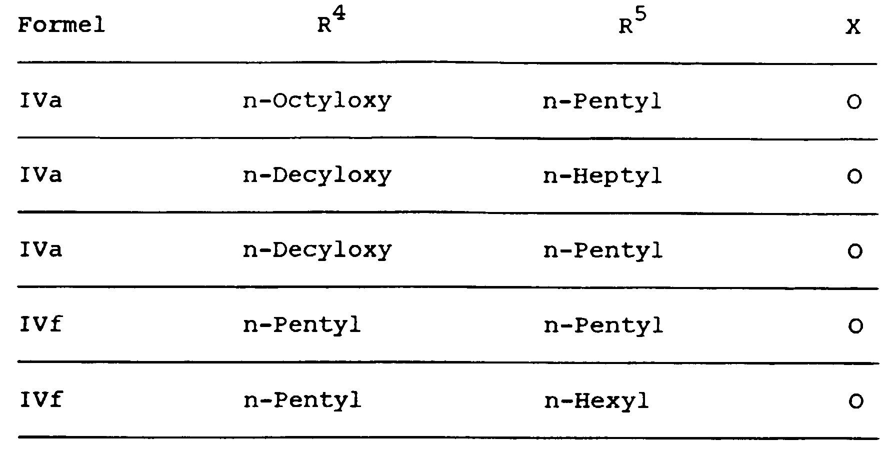

Die Verbindungen der Teilformeln IVc, IVh und IVi eignen sich als Zusätze zur Schmelzpunktserniedrigung und werden normalerweise den Basismischungen mit nicht mehr als 5 %, vorzugsweise 1 bis 3 %, zugesetzt. R⁴ und R⁵ bedeuten in den Verbindungen der Teilformeln IVc, IVh und IVi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel 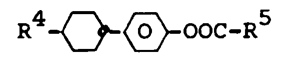 worin R⁴ und R⁵ die für IVc, IVh und IVi angegebene bevorzugte Bedeutung haben.

Als weitere Komponenten mit negativer dielektrischer Anisotropie eignen sich weiterhin Verbindungen enthaltend das Strukturelement A, B oder C. 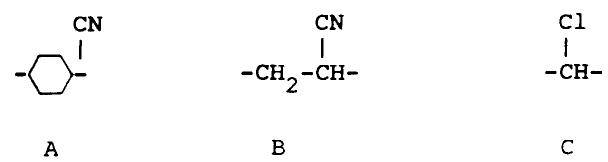 Bevorzugte Verbindungen dieser Art entsprechen den Formeln Va, Vb und Vc:

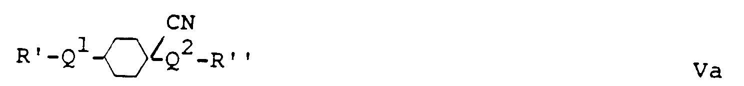

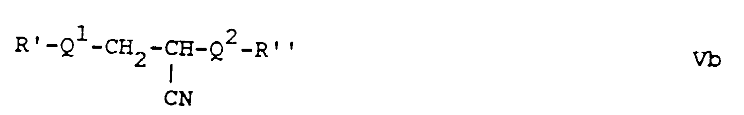

R'-Q³-Q⁴-R''' Vc

R' und R" bedeuten jeweils vorzugsweise geradkettige Alkyl- oder Alkoxy-Gruppen mit jeweils 2 bis 10 C-Atomen. Q¹ und Q² bedeuten jeweils 1,4-Phenylen, trans-1,4-Cyclohexylen, 4,4'-Biphenylyl, 4-(trans-4-Cyclohexyl)-phenyl, trans,trans-4,4'-Bicyclohexyl oder eine der Gruppen Q¹ und Q² auch eine Einfachbindung.

Q³ und Q⁴ bedeuten jeweils 1,4-Phenylen, 4,4'-Biphenylyl oder trans-1,4-Cyclohexylen. Eine der Gruppen Q³ und Q⁴ kann auch 1,4-Phenylen bedeuten, worin mindestens eine CH-Gruppe durch N ersetzt ist. R''' ist ein optisch aktiver Rest mit einem asymmetrischen Kohlenstoffatom der Struktur 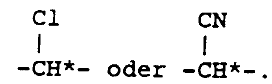 oder Besonders bevorzugte Verbindungen der Formel Vc sind diejenigen der Formel Vc': 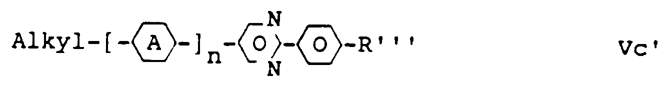 worin A 1,4-Phenylen oder trans-1,4-Cyclohexylen und n 0 oder 1 bedeutet.

Die Verbindungen der Formel I umfassen die nachstehend angeführten bevorzugten zweikernigen und dreikernigen Materialien. 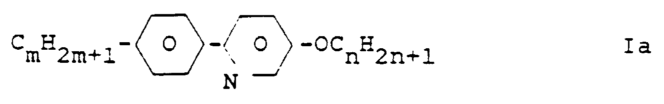 worin m vorzugsweise 7 bis 12 und n 6 bis 12 bedeutet. 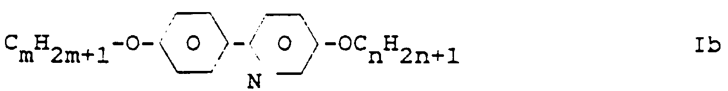 worin n und m vorzugsweise jeweils unabhängig voneinander 5 bis 12 bedeuten. 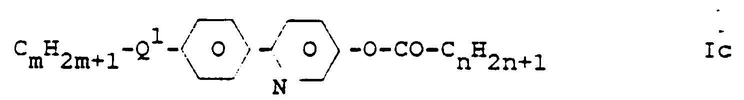 worin der Rest CₙH₂ₙ₊₁ geradkettig ist, und vorzugsweise Q¹ -O- oder eine Einfachbindung und m und n jeweils unabhängig voneinander 5 bis 12 bedeuten. 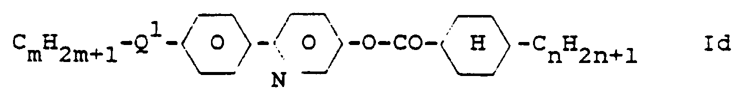 worin vorzugsweise Q¹ -O- oder eine Einfachbindung, m 5 bis 12 und n 2 bis 12 bedeutet. 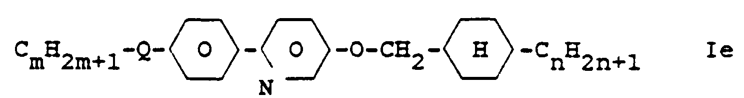 worin vorzugsweise Q -0- oder eine Einfachbindung, m 5 bis 12 und n 2 bis 12 bedeutet. 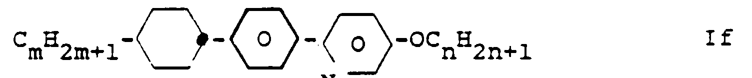 worin m vorzugsweise 1 bis 12 und n 1 bis 12 bedeuten. 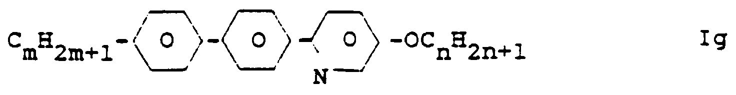 worin m vorzugsweise 3 bis 12 und n 2 bis 12 bedeuten.

Darunter sind diejenigen der Teilformeln Ia und Ib, insbesondere Ib, besonders bevorzugt.

m ist vorzugsweise 5 bis 14, insbesonder 6 bis 12. n ist vorzugsweise 3 bis 12. Die Reste CₘH₂ₘ₊₁ und CₙH₂ₙ₊₁ sind vorzugsweise geradkettig. Verbindungen der Formel I mit relativ kurzen derartigen Resten eignen sich auch als Komponenten nematischer Medien (n und m unabhängig voneinander 1 bis 7).
Q¹ ist vorzugsweise -O-.

Die Verbindungen der Formel I werden nach an sich bekannten Methoden, wie sie in der Literatur (z.B. in den bekannten Standardwerken wie Houben-Weyl, Methoden der Organischen Chemie, Georg-Thieme-Verlag, Stuttgart) beschrieben sind, hergestellt.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die erfindungsgemäßen Verbindungen lassen sich nach folgendem Reaktionsschema einfach herstellen.

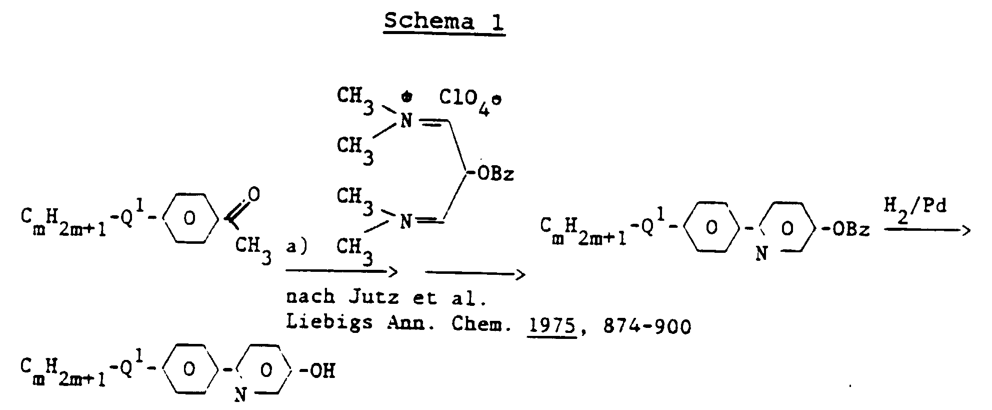

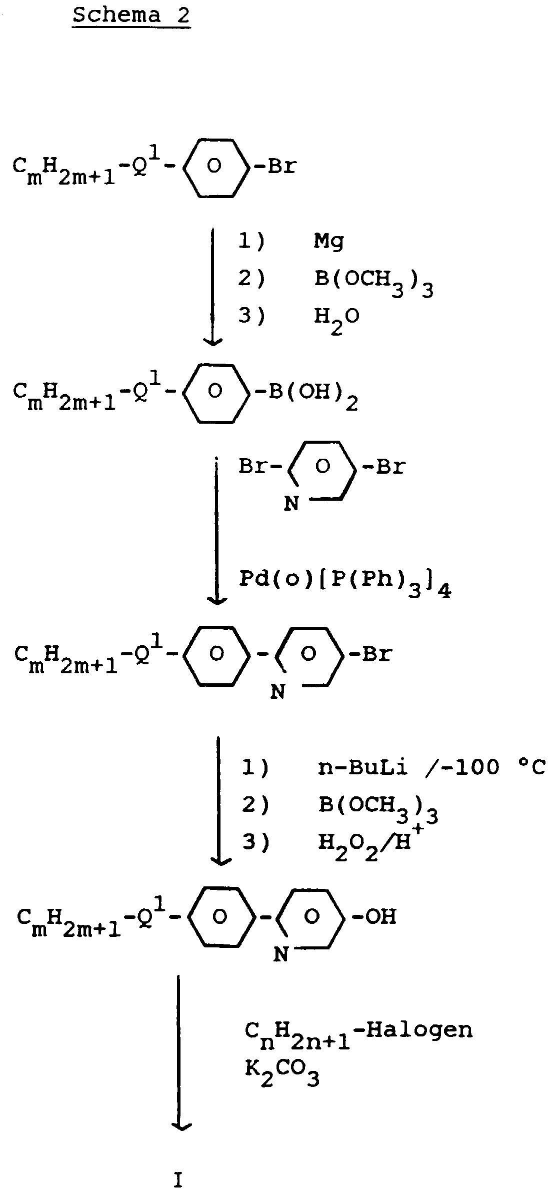

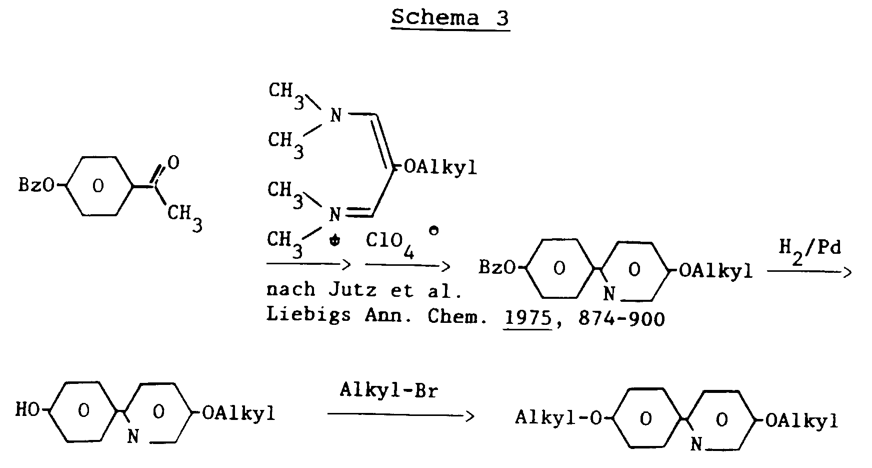

Die erfindungsgemäße Verbindungen sind weiterhin durch Kopplung von metallorganischen Zinkverbindungen mit entsprechenden Brompyridinderivaten entsprechend DE-OS 36 32 410 erhältlich.

Im folgenden wird die Synthese einiger besonders interessanter Hydroxy-Zwischenstufen beschrieben:
a) 5-Hydroxy-2(4-alkylphenyl)pyridine bzw. 5-Hydroxy-2(4-alkoxyphenyl)pyridine sind erhältlich aus 2-Benzyloxytrimethiniumsalz durch Kondensation mit 4-Alkyl- oder 4-Alkoxyacetophenonen, Umsetzung mit NH₃/NH₄Cl oder Ammoniumacetat.
   Analog den Vorschriften von Ch. Jutz et al. (Liebigs Ann. Chem. 1975 874-900) und anschließende Hydrogenolyse oder aus 4-Alkyl- bzw. 4-Alkoxyphenylboronsäure durch Kopplung mit 5-Acetoxy-2-brompyridin (erhältlich aus 5-Hydroxy-2-brompyridin durch Veresterung) in Gegenwart eines Pd-Katalysators entsprechend den Arbeiten von Suzuki et al. (Synth. Commun. 11 513-19 (1981)).
b) 5-Alkoxy-2(4-hydroxyphenyl)pyridine sind erhältlich durch Kopplung von 4-Benzyloxyphenylboronsäure mit 5-Alkoxy-2-brompyridin entsprechend oben genannter Literatur und anschließende Hydrogenolyse.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Rest L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R" voneinander verschieden, wobei einer dieser Reste meist Alkyl oder Alkenyl ist. In den Verbindungen der Teilformeln 1b, 2b, 3b, 4b und 5b bedeutet R" -CN, -CF₃, F, Cl oder -NCS; R hat dabei die bei den Verbindungen der Teil formeln 1a bis 5a angegebene Bedeutung und ist vorzugsweise Alkyl oder Alkenyl. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90 %,
Gruppe 2: 10 bis 80 %, insbesondere 10 bis 50 %,
wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:
K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

### Beispiel 1

0,1 mol 2-(4-nonylphenyl)-5-hydroxypyridin (hergestellt durch Kondensation von 4-Nonylacetophenon mit 2-Benzyloxytrimethiniumperchlorat nach Jutz et al. (siehe Seite 11) mit anschließender Hydrogenolyse des Benzylethers) werden mit 0,11 mol 1-Bromheptan und 0,11 mol Kaliumcarbonat in Dimethylformamid als Lösungsmittel verethert. Nach der Aufarbeitung wird das 2-(4-Nonylphenyl)-5-heptyloxypyridin aus Isopropanol umkristallisiert.

Analog erhält man durch Umsetzung von 4-Benzyloxyacetophenon mit 2-Octyloxytrimethiniumperchlorat, Ammoniumacetat und hydrierende Spaltung des Benzylethers 2-(4-Hydroxyphenyl)-5-octyloxypyridin, das mit 1-Bromdecan zum 2-(4-n-Decyloxyphenyl)-5-octyloxypyridin alkyliert wird.

### Beispiele 44 bis 204:

Analog bzw. durch Veretherung oder Veresterung entsprechender Hydroxyverbindungen nach Standardmethoden werden folgende Verbindungen der Formel I hergestellt:

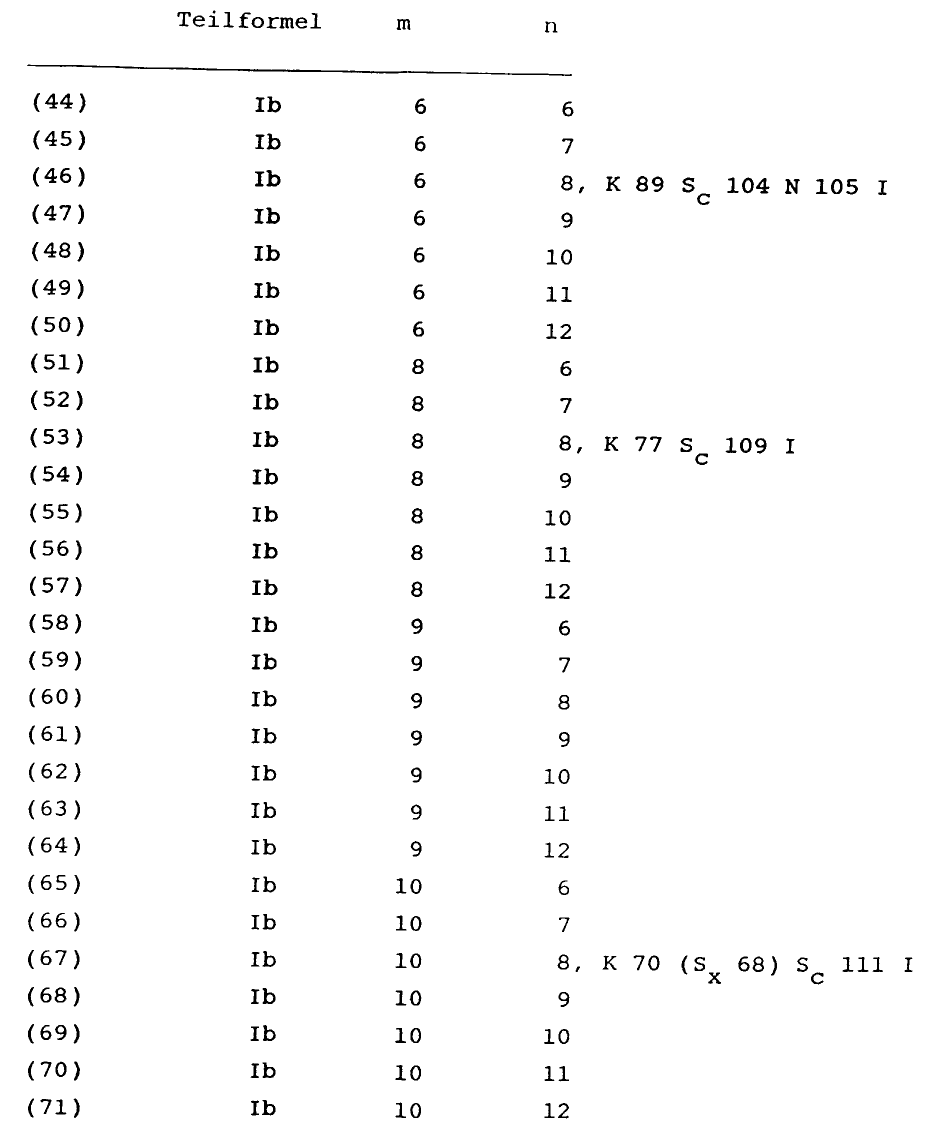

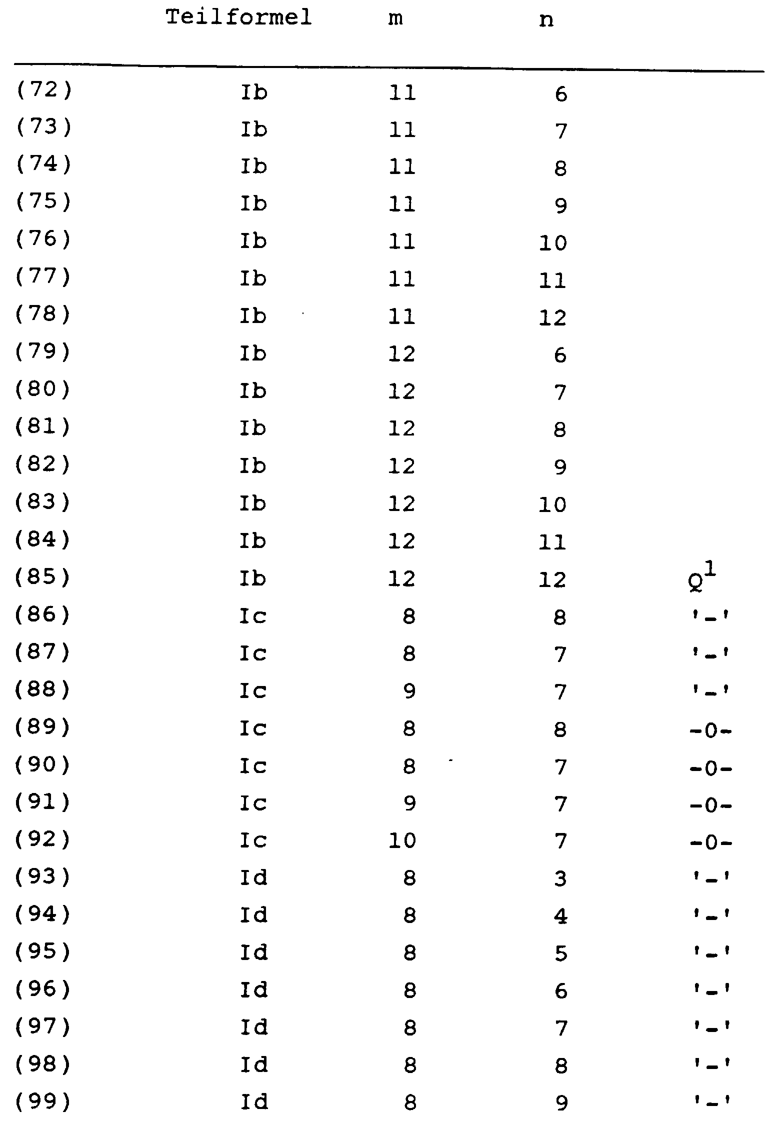

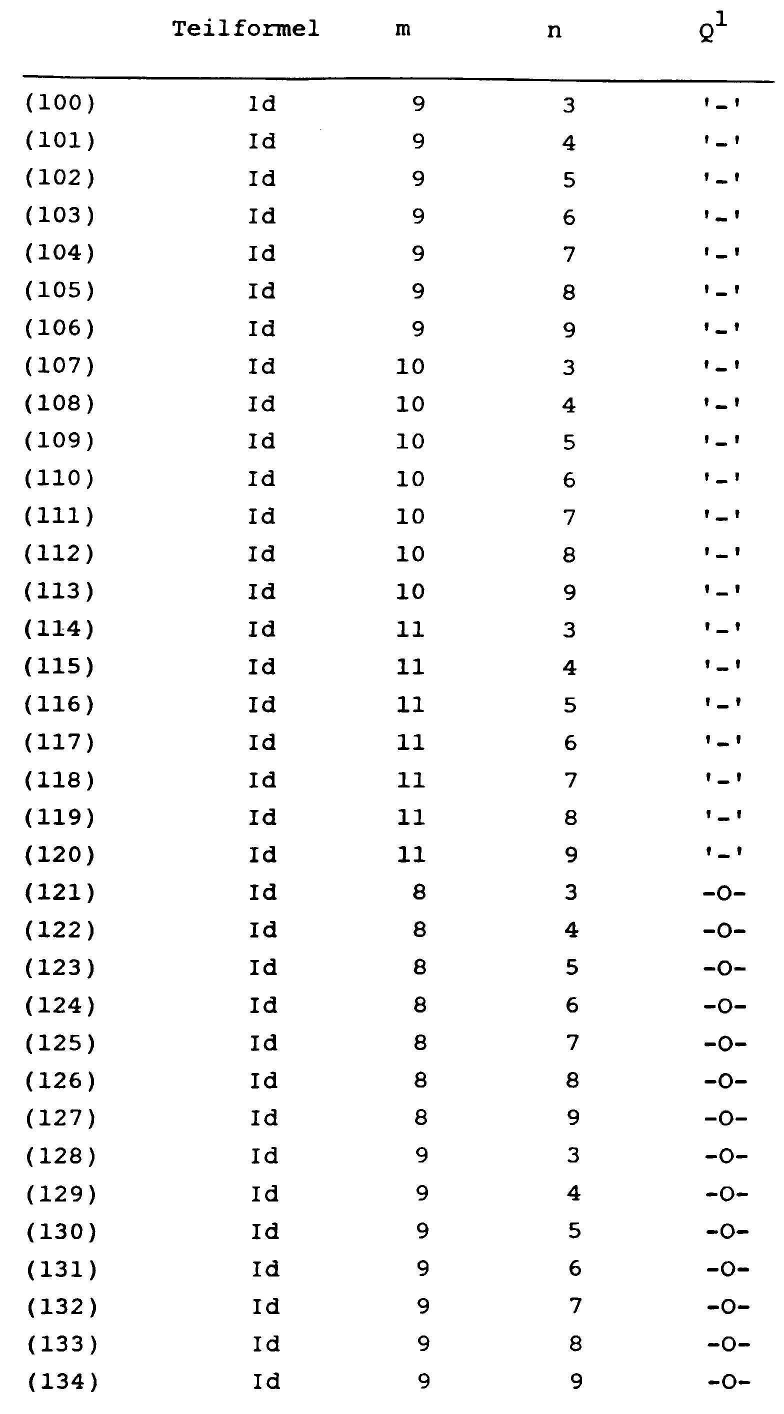

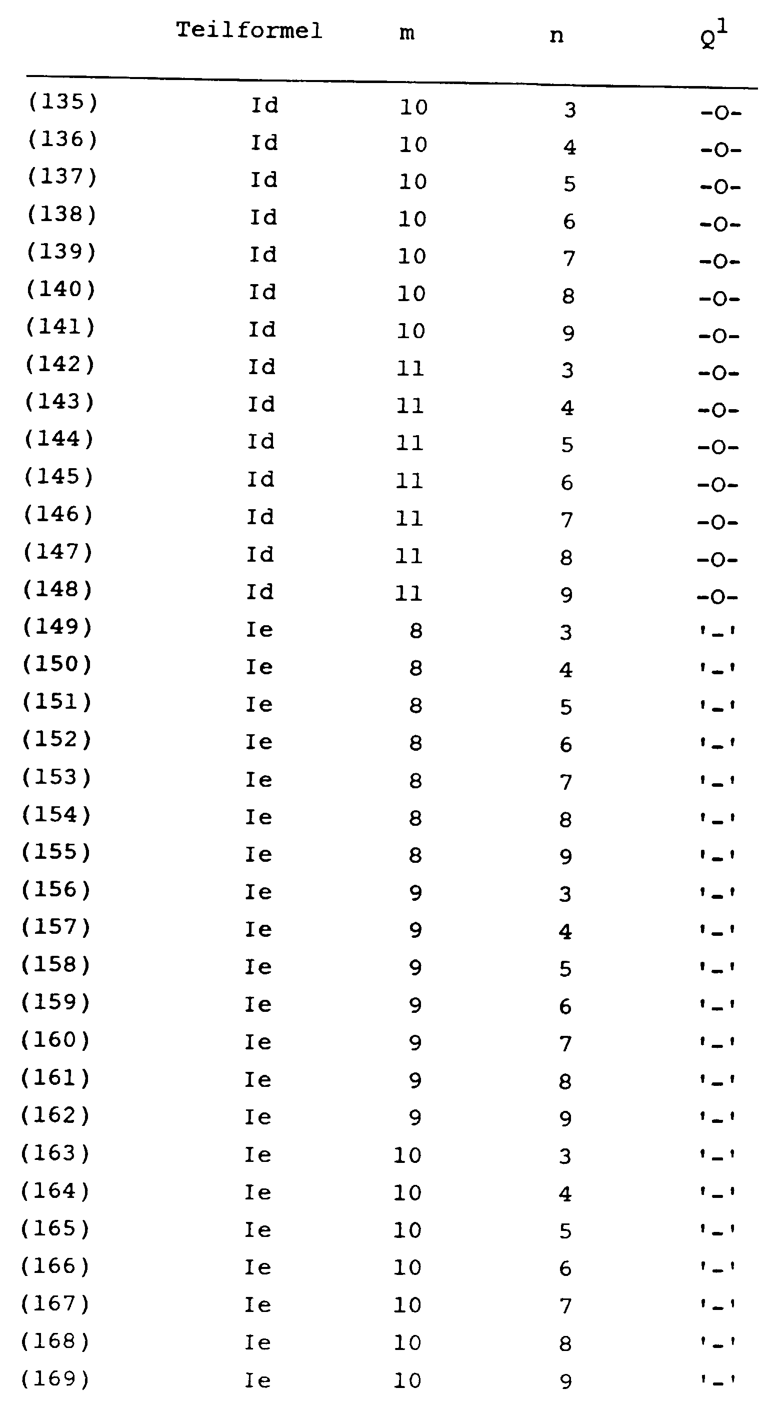

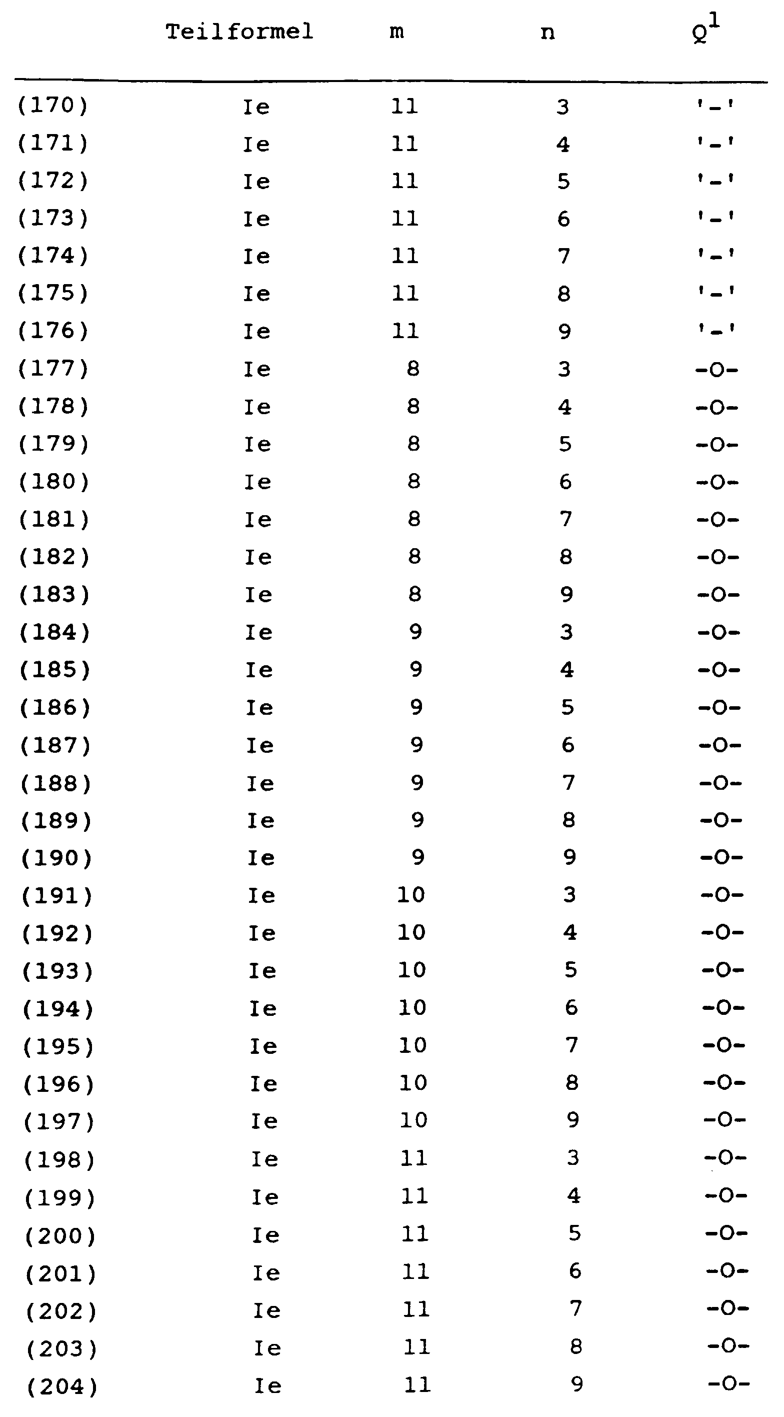

### Beispiel 205

0,1 mol 4-(trans-4-Pentylcyclohexyl)-phenyl-boronsäure werden mit 0,1 mol 2,5-Dibrompyridin in Toluol/2n-NaHCO₃-Lösung (1:1) mit tetrakis [Triphenylphosphin] Palladium (0) als Katalysator gemäß Schema 2 gekoppelt. Das Kopplungsprodukt wird bei -100 °C in THF mit n-Butyllithium und anschließend mit Trimethylborat versetzt. Die so erhaltene Boronsäure wird mit 20 ml einer 3%igen H₂O₂-Lösung versetzt und 2 hrs bei Raumtemperatur gerührt. Das erhaltene 2-[4-(trans-4-Pentylcyclohexyl)-phenyl]-5-hydroxypyridin wird analog Beispiel 1 mit 1-Bromoctan verethert. Nach der Aufarbeitung wird das 2-[4-(trans-4-Pentylcyclohexyl)-phenyl]-5-octyloxypyridin umkristallisiert.

### Beispiele 206 bis 223

Analog werde folgende Verbindungen der Formel I hergestellt

| Beispiel | Teilformel | m | n |
|---|---|---|---|
| (206) | If | 3 | 8 |
| (207) | If | 3 | 9 |
| (208) | If | 3 | 10 |
| (209) | If | 3 | 11 |
| (210) | If | 5 | 2, K 94 N 222 I |
| (211) | If | 5 | 9 |
| (212) | If | 5 | 10 |
| (213) | If | 7 | 8 |
| (214) | If | 7 | 9 |
| (215) | If | 7 | 10 |
| (216) | If | 8 | 8 |
| (217) | If | 8 | 9 |
| (218) | If | 8 | 10 |
| (219) | If | 8 | 6 |
| (220) | Ig | 8 | 8 |
| (221) | Ig | 7 | 8 |
| (222) | Ig | 8 | 6 |
| (223) | Ig | 10 | 10 |

## Patentansprüche

1. Ferroelektrisches flüssigkristallines Medium mit mindestens zwei flüssigkristallinen Komponenten, dadurch gekennzeichnet, daß es ein oder mehrere 5-Oxy-2-phenlpyridine der Formel I enthält, 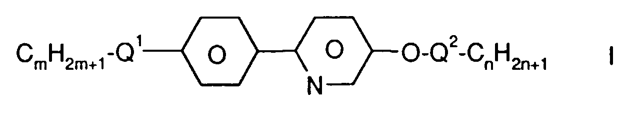 worin
m und n jeweils unabhängig voneinander 1 bis 18,
Q¹ -O-, trans-1,4-Cyclohexylen, 1,4-Phenylen oder eine Einfachbindung und
Q² 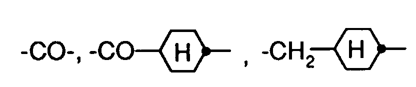 oder eine Einfachbindung bedeutet,
mit den Maßgaben, daß a) im Falle Q¹ = Q² = Einfachbindung die Summe von m + n ≥ 11 ist, und b) im Falle Q² = CO der Rest -CₙH₂ₙ₊₁ geradkettig ist.

2. 5-Oxy-2-phenylpyridine gekennzeichnet durch die Formel Ib 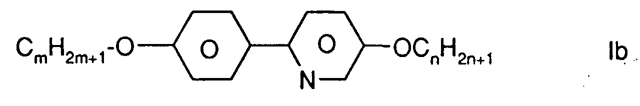 worin n und m jeweils unabhängig voneinander 5 bis 12 bedeuten.

3. 5-Oxy-2-phenylpyridine gekennzeichnet durch die Formel Ic 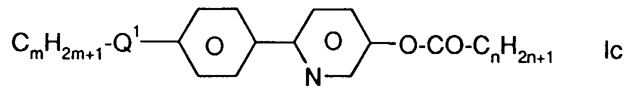 worin der Rest CₙH₂ₙ₊₁ geradkettig ist, und Q¹ -O- oder eine Einfachbindung und m und n jeweils unabhängig voneinander 5 bis 12 bedeuten.

4. 5-Oxy-2-phenylpyridine gekennzeichnet durch die Formel Id 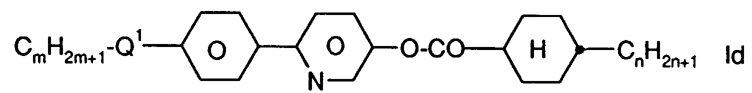 worin Q¹ -O- oder eine Einfachbindung, m 5 bis 12 und n 2 bis 12 bedeutet.

5. 5-Oxy-2-phenylpyridine gekennzeichnet durch die Formel le 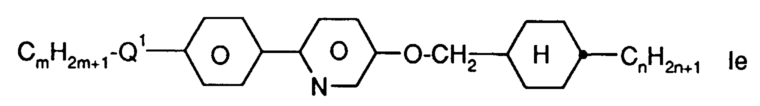 worin Q¹ -O- oder eine Einfachbindung, m 5 bis 12 und n 2 bis 12 bedeutet.

6. 5-Oxy-2-phenylpyridine der Formel If 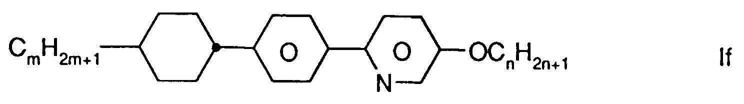 worin m und n jeweils unabhängig voneinander 1 bis 12 bedeuten.

7. Elektrooptische Anzeige, dadurch gekennzeichnet, daß sie als Dielektrikum ein Medium nach Anspruch 1 enthält.

## Claims

1. A ferroelectric liquid-crystalline medium having at least two liquid-crystalline components, which contains one or more 5-oxy-2-phenylpyridines of the formula I 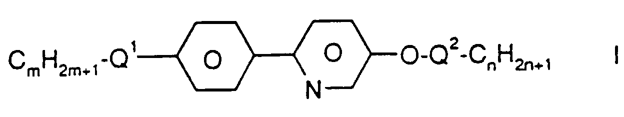 in which
m and n, independently of one another, are each from 1 to 18,
Q¹ is -O-, trans-1,4-cyclohexylene, 1,4-phenylene or a single bond, and
Q² is -CO-, 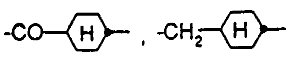 or a single bond,
with the provisos that a) in the case where Q¹ = Q² = a single bond, the sum of m + n ≥ 11, and b) in the case where Q² = CO, the radical -CₙH₂ₙ₊₁ is straight-chain.

2. A 5-oxy-2-phenylpyridine which has the formula Ib 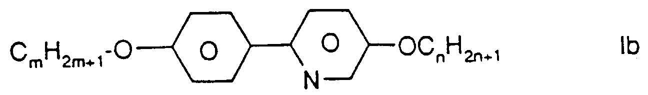 in which n and m, independently of one another, are each from 5 to 12.

3. A 5-oxy-2-phenylpyridine which has the formula Ic 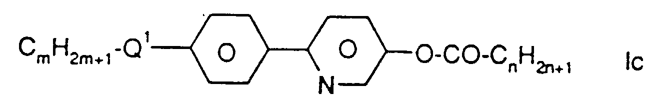 in which the radical CₙH₂ₙ₊₁ is straight-chain, and Q¹ is -O- or a single bond, and m and n, independently of one another, are each from 5 to 12.

4. A 5-oxy-2-phenylpyridine which has the formula Id 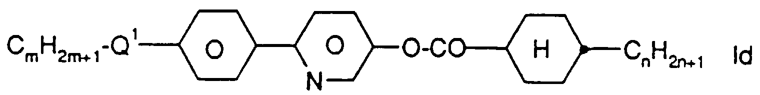 in which Q¹ is -O- or a single bond, m is from 5 to 12 and n is from 2 to 12.

5. A 5-oxy-2-phenylpyridine which has the formula Ie 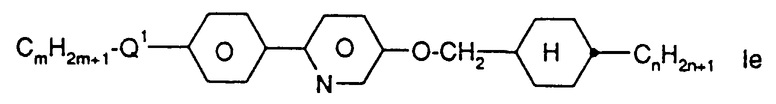 in which Q¹ is -O- or a single bond, m is from 5 to 12 and n is from 2 to 12.

6. A 5-oxy-2-phenylpyridine of the formula If 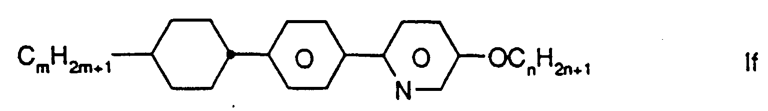 in which m and n, independently of one another, are each from 1 to 12.

7. An electrooptical display, which contains, as dielectric, a medium as claimed in claim 1.

## Revendications

1. Milieu à cristaux liquides ferroélectriques avec au moins deux composants de cristaux liquides, caractérisé en ce qu'il contient un ou plusieurs 5-oxy-2-phénylpyridines de formule I, 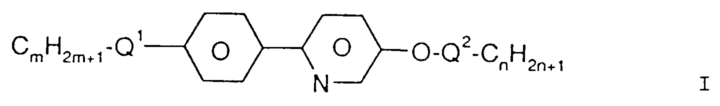 dans laquelle
m et n chacun indépendamment l'un de l'autre va de 1 à 18,
Q¹ représente -O-, trans-1,4-cyclohexylène, 1,4-phénylène ou une liaison simple et
Q² représente -CO-, 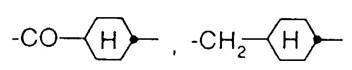 ou une simple liaison,
à la condition que a) dans le cas où Q¹ = Q² = liaison simple, la somme de m + n ≥ 11, et b) dans le cas où Q² = CO, le radical -CₙH₂ₙ₊₁ soit linéaire.

2. 5-Oxy-2-phénylpyridines caractérisés par la formule Ib 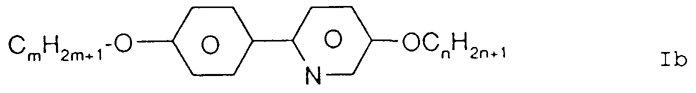 dans laquelle n et m chacun, indépendamment l'un de l'autre, va de 5 à 12.

3. 5-Oxy-2-phénylpyridines caractérisés par la formule Ic 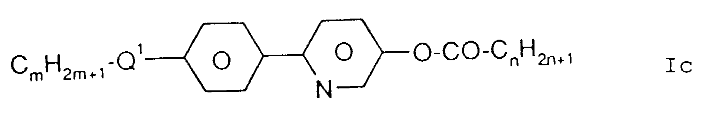 dans laquelle le radical CₙH₂ₙ₊₁ est linéaire et Q¹ représente -O- ou une liaison simple et m et n chacun, indépendamment l'un de l'autre, va de 5 à 12.

4. 5-Oxy-2-phénylpyridines caractérisés par la formule Id 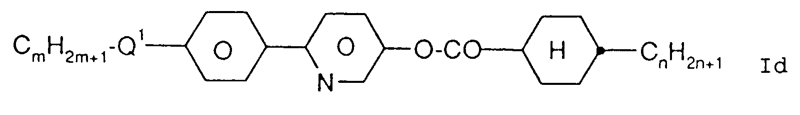 dans laquelle Q¹ représente -O- ou une liaison simple, m va de 5 à 12 et n va de 2 à 12.

5. 5-Oxy-2-phénylpyridines caractérisés par la formule Ie 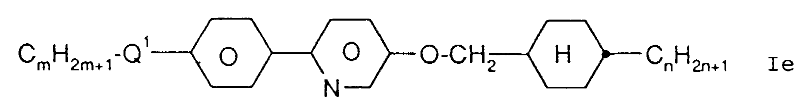 dans laquelle Q¹ représente -O- ou une liaison simple, m va de 5 à 12 et n va de 2 à 12.

6. 5-Oxy-2-phénylpyridines caractérisés par la formule If 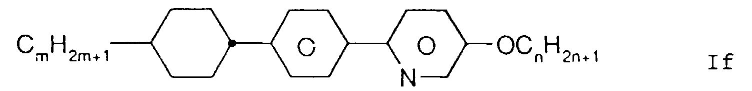 dans laquelle m et n chacun, indépendamment l'un de l'autre, va de 1 à 12.

7. Affichage électro-optique caractérisé en ce qu'il contient comme diélectrique un milieu selon la revendication 1.
